# EUROPEAN PATENT SPECIFICATION

(11) **EP 1 510 588 B1**
(45) Date of publication and mention of the grant of the patent: **31.01.2007**
(21) Application number: 04020100.6
(22) Date of filing: 25.08.2004
(51) Int. Cl.: C12Q 1/68

(54) **Correlation of the ratio of thymidine phosphorylase and dihydropyrimidine dehydrogenase mRNA expression levels in colorectal cancer with disease-free survival in 5-FU treated patients**
Abhängigkeit des symptomfreien Überlebens 5-FU behandelter Darmkrebspatienten vom Verhältnis der mRNA-Expressionshöhe aus Thymidinphosphorylase und Dihydropyrimidindehydrogenase
Corrélation de ratio de l'expression d'ARNm de thymidine phosphorylase et dihydropyrimidine déshydrogénase avec la survie maladie-libre dans les patients atteints de cancer de l'intestin traités avec 5-FU

(30) Priority: 28.08.2003 EP 03018783
(43) Date of publication of application: 02.03.2005
(73) Proprietor: Roche Diagnostics GmbH, 68305 Mannheim (DE); F. HOFFMANN-LA ROCHE AG, 4070 Basel (CH)
(72) Inventor: Lutz, Verena, Dr., 81475 München (DE); Krause, Friedmann, Dr., 82377 Penzberg (DE); Poignee, Manuela, Dr., 83623 Dietramszell/Schoenegg (DE); Walter, Thomas, Dr., 82377 Penzberg (DE); Porstmann, Baerbel, Prof. Dr., 67134 Birkenheide (DE); Werner, Martin, Prof. Dr., 79104 Freiburg (DE); Lassmann, Silke, Dr., 79104 Freiburg (DE)

(56) References cited:
- WO-A-02/070750
- TERASHIMA M ET AL: "Role of thymidine phosphorylase and dihydropyrimidine dehydrogenase in tumour progression and sensitivity to doxifluridine in gastric cancer patients" EUROPEAN JOURNAL OF CANCER, PERGAMON PRESS, OXFORD, GB, vol. 38, no. 18, December 2002 (2002-12), pages 2375-2381, XP004395675 ISSN: 0959-8049
- FUJIWAKI R ET AL: "Gene expression for dihydropyrimidine dehydrogenase and thymidine phosphorylase influences outcome in epithelial ovarian cancer." JOURNAL OF CLINICAL ONCOLOGY: OFFICIAL JOURNAL OF THE AMERICAN SOCIETY OF CLINICAL ONCOLOGY. UNITED STATES 1 DEC 2000, vol. 18, no. 23, 1 December 2000 (2000-12-01), pages 3946-3951, XP008026200 ISSN: 0732-183X
- SALONGA D ET AL: "Colorectal tumors responding to 5-fluorouracil have low gene expression levels of dihydropyrimidine dehydrogenase, thymidylate synthase, and thymidine phosphorylase" CLINICAL CANCER RESEARCH, THE AMERICAN ASSOCIATION FOR CANCER RESEARCH, US, vol. 6, no. 4, April 2000 (2000-04), pages 1322-1327, XP002245518 ISSN: 1078-0432
- ISHIKAWA T ET AL: "Positive correlation between the efficacy of capecitabine and doxifluridine and the ratio of thymidine phosphorylase to dihydropyrimidine dehydrogenase activities in tumors in human cancer xenografts." CANCER RESEARCH. UNITED STATES 15 FEB 1998, vol. 58, no. 4, 15 February 1998 (1998-02-15), pages 685-690, XP008025976 ISSN: 0008-5472
- COLLIE-DUGUID ELAINA S R ET AL: "Thymidine phosphorylase and dihydropyrimidine dehydrogenase protein expression in colorectal cancer" INTERNATIONAL JOURNAL OF CANCER, vol. 94, no. 2, 15 October 2001 (2001-10-15), pages 297-301, XP002266542 ISSN: 0020-7136
- HIRANO YASUHIRO ET AL: "Thymidine phosphorylase and dihydropyrimidine dehydrogenase in renal cell carcinoma: Relationship between histological parameters and chemosensitivity to 5-fluorouracil." EUROPEAN UROLOGY, vol. 43, no. 1, January 2003 (2003-01), pages 45-52, XP001184101 ISSN: 0302-2838 (ISSN print)

## Description

### Technical field

The present invention relates to the field of prediction of susceptability for drug cancer treatment using biomarker analysis. In particular, the present invention relates to the field of prediction of susceptability of patients suffering from colorectal cancer for 5-FU and/or 5-FU analoga.

### Prior art background

Colorectal cancer (CRC), which remains one of the major death causing cancers in the western world, can be cured in about one-third of patients by surgical resection of the primary tumor alone. However, the remaining patients will already have developed occult or distant metastasis at the time of clinical manifestation of the primary tumor and will receive adjuvant (post-surgery) chemo- and/or radiotherapy. Together with the patient's clinical data, histopathological classification of the tumor and staging according to the TNM categories define the criteria for adjuvant therapy.

As the current standard, nodal negative tumors (T1-4, N0, M0) are treated by complete resection of the primary tumor alone, whereas cases with histopathological evidence of lymph node involvement (T1-4, N1-2, M0) will receive chemo- and/or radiotherapy after surgical removal of the primary tumor. Despite a constant improvement of diagnostic tools and relevant markers as well as treatment strategies [Ragnhammar, P., et al., Acta Oncol. 40 (2001) 282-308], precise prognosis and/or response prediction to adjuvant therapy is still an unsolved issue [Iqbal, S., and Lenz, H.J., Curr. Oncol. Rep. 3 (2001) 102-108; Kumar, S.K., and Goldberg, R.M., Curr. Oncol. Rep. 3 (2001) 94-101].

The enzymes thymidine phosphorylase (TP), dihydropyrimidine dehydrogenase (DPD) and thymidylate synthase (TS) are involved in the metabolism of pyrimidines, and hence eventually involved in proliferation of normal as well as pathologically transformed cells. TS is the central and limiting enzyme for *de novo* synthesis of pyrimidines and therefore also for RNA/DNA synthesis, DPD is involved in the degradation of uracil and thymine to inactive waste products and TP controls intracellular thymidine levels [Diasio, R.B., and Johnson, M.R., Pharmacology 61 (2000) 199-203]. Moreover, TP was shown to be identical to the molecule platelet-derived endothelial cell growth factor (PD-ECGF) [Furukawa, T., et al., Nature 356 (1992) 668; Sumizawa, T., et al., J. Biochem. 114 (1993) 9-14] and exhibits angiogenic properties in a number of solid tumors [Takebayashi, Y., et al., J. Natl. Cancer Inst. 88 (1996) 1110-1117; Griffiths, L, and Stratford, I.J., Br. J. Cancer 76 (1997) 689-693]. These properties may render TP, DPD and /or TS expression valuable prognostic and chemopredictive markers in CRC.

Furthermore, due to their involvement in pyrimidine metabolism, all three enzymes are also important for the efficacy of 5-FU and 5-FU related chemotherapeutic agents [Diasio, R.B., and Johnson, M.R., Pharmacology 61 (2000) 199-203; WO 02/44423]. Whereas TS is the main target of such chemotherapeutics, the enzymes TP and DPD function in the activation and degradation of these agents and their intermediates, respectively. As such, the three enzymes, individually or the TS/DPD and TP/DPD ratios, have been implicated as markers for prognosis and/or response prediction to adjuvant chemotherapy in CRC and other cancer types [Metzger, R., et al., Clin. Cancer Res. 4 (1998) 2371-2376; Salonga, D., et al., Clin. Cancer Res. 6 (2000) 1322-1327; Takenoue, T., et al., Ann. Surg. Oncol. 7 (2000) 193-198; Araki, Y., et al., Kurume Med. J. 48 (2001) 93-98; Edler, D., et al., J. Clin. Oncol. 20 (2002) 1721-1728; Kornmann, M., et al., J. Gastrointest. Surg. 6 (2002) 331-337]. Terashima M. et al, Europ. J of Cancer (2002) 2375-2381; Fujwaki R. et .al, J of Clinical Oncology (2000) 3946-3951. In fact, high TS levels have been linked to the resistance of tumors to 5-FU chemotherapy [Copur, S., et al., Biochem. Pharmacol. 49 (1995) 1419-1426; Wang, W., et al., Cancer Res. 61 (2001) 5505-5510; Johnston, P.G., et al., Cancer Res. 55 (1995) 1407-1412; Bathe, O.F., et al., Cancer J. Sci. Am. 5 (1999) 34-40] and low DPD expression has been correlated to severe toxicity to 5-FU chemotherapeutic agents [Wei, X., et al., J. Clin. Invest. 98 (1996) 610-615, Johnson, M.R., et al., Clin. Cancer Res. 5 (1999) 2006-2011]. The wide range of TS and DPD expression and the associated effects on chemotherapy outcome appear to be, at least in part, due to a polymorphism in the TS promotor enhancer region [Marsh, S., et al., Int. J. Oncol. 19 (2001) 383-386; Iacopetta, B., et al., Br. J. Cancer 85 (2001) 827-830] and a common point mutation within intron 14 of the DPD gene [van Kuilenburg, A.B., et al., Clin. Cancer Res. 6 (2000) 4705-4712; Raida, M., et al., Clin. Cancer Res. 7 (2001) 2832-2839], respectively. Despite a number of studies examining TP, DPD and TS expression in CRC, the role of the enzymes is still conflicting. This is mainly due to diverse study protocols, analysing TP, DPD and TS expression with respect to either 1) mRNA, protein or enzyme activity, 2) in primary tumors or distant metastases and 3) patients treated with various protocols of neo- or adjuvant radio- / chemotherapy.

In view of the outlined prior art, the problem to be solved was the identification of those parameters, which of all potential parameters suggested in the prior art actually are indicative for susceptability to 5-FU and/or 5-FU analoga and to determine, under which conditions these parameter(s) provide for the highest possible specificity.

### Brief description of the invention

Thus, the present invention is directed to a method for determining whether a patient suffering from colorectal cancer is susceptable to a treatment with 5-Fluoro-Uracil or a 5-Fluoro-Uracil analogon comprising
a) determination of Thymidine Phosphorylase mRNA expression in a clinical sample
b) determination of Dihydropyrimidine Dehydrogenase mRNA expression in said clinical sample
c) determination of the ratio of the value obtained in step a) and the value obtained in step b)
d) determination whether the ratio obtained in step c exceeds a predetermined cut off value

In order to avoid too many false positve results, it has been proven to be advantageous, if said cut off value for TP/DPD ratio is at least 3 and preferably 3.7.

On the other hand, in order to avoid too many false negative results, it has been proven to be advantageous, if said cut off value for TP/DPD ratio is not higher than 10 and preferably not higher than 8.2.

In a particular embodiment, there is an additional determination of the absolute or relative expression level of Dihydropyrimidine Dehydrogenase and expression value below a certain cut off value is additionally indicative for susceptablity to 5 Fluoro-Uracil or a respective analogon.

### Detailed description of the invention

In the study underlying the invention, TP, DPD and TS mRNA expression was examined in a unique group of 102 patients with CRC, using microdissected, formalin-fixed and paraffin-embedded primary tumor samples for quantitative RT-PCR (QRT-PCR) in the LightCycler^{®} system. We specifically address the correlation of TP, DPD and TS mRNA expression and the TS/DPD and TP/DPD ratios to tumor histology as well as to patient prognosis and response prediction to 5-FU adjuvant chemotherapy.

The inventors investigated in detail thymidine phosphorylase (TP), dihydropyrimidine dehydrogenase (DPD) und thymidilate synthase (TS) mRNA expression in CRC with emphasis on their value as prognostic markers in general and as predictive markers for 5-FU chemotherapy. For this, a novel approach of TP, DPD and TS mRNA quantification, RT-PCR using the LightCycler^{®} system (Roche Applied Science), was developed and applied to microdissected, formalin-fixed, paraffin-embedded tumor tissues of 102 cases with CRC. In contrast to other studies [Johnston, P.G., et al., Cancer Res. 55 (1995) 1407-1412] [Metzger, R., et al., Clin. Cancer Res. 4 (1998) 2371-2376; Salonga, D., et al., Clin. Cancer Res. 6 (2000) 1322-1327], the inventors retrospectively examined TP, DPD and TS mRNA expression in primary tumors and evaluated the progostic impact and clinical response of patients to 5-FU chemotherapy by correlation of enzyme levels to patient follow up. Although TP, DPD and / or TS expression have been analysed in primary CRC tumors with respect to prognosis [Takebayashi, Y., et al., J. Natl. Cancer Inst. 88 (1996) 1110-1117; Araki, Y., et al., Kurume Med. J. 48 (2001) 93-98; Edler, D., et al., J. Clin. Oncol. 20 (2002) 1721-1728; Sanguedolce, R., et al., Anticancer Res. 18 (1998) 1515-1520; Paradiso, A., et al., Br. J. Cancer 82 (2000) 560-567; Findlay, M.P., et al., Br. J. Cancer 75 (1997) 903-909; Allegra, C.J., et al., J. Clin. Oncol. 20 (2002) 1735-1743], our study is the first to examine the prognostic and predictive value of TP, DPD and TS mRNA expression in a group of CRC cases of unique sample size, treatment sub-groups, follow-up data and standardised tissue sampling and preservation.

Detailed analysis of TP, DPD and TS mRNA expression in 102 cases of CRC revealed a wide range of expression levels for all three enzymes. This has been reported before [Iqbal, S., and Lenz, H.J., Curr. Oncol. Rep. 3 (2001) 102-108; Metzger, R., et al., Clin. Cancer Res. 4 (1998) 2371-2376; Mori, K., et al., Int. J. Oncol. 17 (2000) 33-38] and together these results underline the concept of intertumor heterogeneity.

Finally, the association of TP and TS mRNA levels with a particular tumor histopathology were also reflected by the TP/DPD and TS/DPD ratios. One explanation for these findings may relate to the biological functions of these enzymes: Whereas TP, also known as platelet-derived endothelial cell growth factor [Furukawa, T., et al., Nature 356 (1992) 668; Sumizawa, T., et al., J. Biochem. 114 (1993) 9-14], is associated with angiogenesis in a number of solid tumors [Takebayashi, Y., et al., J. Natl. Cancer Inst. 88 (1996) 1110-1117; Griffiths, L, and Stratford, I.J., Br. J. Cancer 76 (1997) 689-693], TS may be regarded as a marker of metabolic activity and cellular proliferation [Pestalozzi, B.C., et al., Br. J. Cancer 71 (1995) 1151-1157; Backus, H.H., et al., J. Clin. Pathol. 55 (2002) 206-211; Pestalozzi, B.C., et al., Br. J. Cancer 71 (1995) 1151-1157]. Therefore, higher TP and TS mRNA expression in "early" tumors may reflect their activity in promoting vascular support and tumor cell proliferation, which is reduced upon progression to favour, for example, tumor cell invasion and metastasis. In fact, low levels of TP, DPD and TS mRNA expression levels have been associated with a favourable response to 5-FU chemotherapy [Metzger, R., et al., Clin. Cancer Res. 4 (1998) 2371-2376; Salonga, D., et al., Clin. Cancer Res. 6 (2000) 1322-1327]. Indeed, also from our data it is exactly this group of tumors with progressed UICC stages, i.e. those patients receiving adjuvant chemotherapy, which express lower TP and TS mRNA levels.

Previous reports have discussed TP, DPD and TS mRNA expression, alone or in combination, as potential markers for prognosis [Takenoue, T., et al., Ann. Surg. Oncol. 7 (2000) 193-198; Edler, D., et al., J. Clin. Oncol. 20 (2002) 1721-1728; Sanguedolce, R., et al., Anticancer Res. 18 (1998) 1515-1520; Paradiso, A., et al., Br. J. Cancer 82 (2000) 560-567; Allegra, C.J., et al., J. Clin. Oncol. 20 (2002) 1735-1743] and / or response prediction to 5-FU chemotherapy [Metzger, R., et al., Clin. Cancer Res. 4 (1998) 2371-2376; Salonga, D., et al., Clin. Cancer Res. 6 (2000) 1322-1327; Araki, Y., et al., Kurume Med. J. 48 (2001) 93-98; Johnston, P.G., et al., Cancer Res. 55 (1995) 1407-1412] in CRC. In addition, the TP/DPD and / or TS/DPD ratios have recently been implicated as prognostic and / or predictive markers [Kornmann, M., et al., J. Gastrointest. Surg. 6 (2002) 331-337; Ishikawa, T., et al., Cancer Res. 58 (1998) 685-690. Terashima M. et al, Europ. J. of Cancer (2002), 2375-2381; Fujiwaki R. et al, J of Clinical Oncology (2000) 3946-3951. In the present study, the inventors could identify the TS/DPD ratio as a potential prognostic marker, with higher TS/DPD ratios correlating with poorer overall survival in CRC patients receiving resection alone.

More important, the present study revealed a significant correlation of DPD mRNA levels and the TP/DPD ratio with disease-free survival after 5-FU chemotherapy, whereby low DPD mRNA levels and a high TP/DPD ratio predict for a longer disease-free survival. This may be related to the fact that low DPD levels alone or low DPD levels and high TP levels (high TP/DPD ratio) will stabilize the active level of 5-FU. In contrast, neither TP, DPD or TS mRNA levels or the TP/DPD or TS/DPD ratio had any predictive value for overall survival.

Whereas previous studies have addressed TP, DPD or TS protein expression in primary CRC tumors by immunohistochemistry [Takebayashi, Y., et al., J. Natl. Cancer Inst. 88 (1996) 1110-1117; Edler, D., et al., J. Clin. Oncol. 20 (2002) 1721-1728; Paradiso, A., et al., Br. J. Cancer 82 (2000) 560-567; Findlay, M.P., et al., Br. J. Cancer 75 (1997) 903-909; Allegra, C.J., et al., J. Clin. Oncol. 20 (2002) 1735-1743], protein content [Sanguedolce, R., et al., Anticancer Res. 18 (1998) 1515-1520] or enzyme activity [Araki, Y., et al., Kurume Med. J. 48 (2001) 93-98], the inventors decided on TP, DPD and TS mRNA analysis by quantitative RT-PCR, as this method is fast, reliable, easy to standardize and works well for large series of formalin fixed, paraffin embedded, microdissected tissue samples. A difficulty to detect any prognostic and / or predictive value of these enzymes on the RNA level might have been expected, due to post-transcriptional, fixation related (and associated functional) modifications [Kawakami, K., et al., Clin. Cancer Res. 7 (2001) 4096-4101].

However, several investigators have shown a good correlation of, for example, DPD and TS gene and protein expression [Johnston, P.G., et al., Cancer Res. 55 (1995) 1407-1412; Uetake, H., et al., Clin. Cancer Res. 5 (1999) 2836-2839; Tanaka-Nozaki, M., et al., Clin. Cancer Res. 7 (2001) 2783-2787]. In order to validate our approach, the inventors initially screened microdissected cells from control tissues. The results did reflect previously published data, with, for example, high TP levels in inflammatory cells [Fox, S.B., et al., J. Pathol. 176 (1995) 183-190] and high DPD levels in the liver [Guimbaud, R., et al., Cancer Chemother. Pharmacol. 45 (2000) 477-482; Johnston, S.J., et al., Clin. Cancer Res. 5 (1999) 2566-2570]. Furthermore, tumors were reported to have lower DPD [Johnston, S.J., et al., Clin. Cancer Res. 5 (1999) 2566-2570; Miyamoto, S., et al., Int. J. Oncol. 18 (2001) 705-713] and higher TP [Takebayashi, Y., et al., Eur. J. Cancer 32 (1996) 1227-1232; Miwa, M., et al., Eur. J. Cancer 34 (1998) 1274-1281] levels than "normal" tissues, a concept underlying tumor-specificity for 5-FU pro-drugs.

Whereas the inventors also detected lower DPD levels in the "tumor" cell than "normal epithelial" cell isolates, the results did not reveal differences between TP and TS mRNA levels in tumor and "normal" cells. This most likely reflects the fact of how precisely one defines a "normal" cell population. For interpretation of TP, DPD and TS expresssion, morphologically controlled tissue acquisition is important, as non-epithelial cells may significantly influence the results. Thus, when the inventors compared "normal colonic smooth muscle cells" (as opposed to the "normal epithelial cell" samples) with "tumor" cells, the latter exhibited higher TP and TS mRNA expression, as previously reported [Takebayashi, Y., et al., Eur. J. Cancer 32 (1996) 1227-1232; Miwa, M., et al., Eur. J. Cancer 34 (1998) 1274-1281].

In summary, the present invention is directed to a novel, quantitative RT-PCR approach for determing 5-FU and/or 5-FU analogon susceptability in colorectal cancer patients. The new method is characterized in that determination of the TP/DPD ratio and optionally DPD mRNA expression in FFPE biopsies from patients. TP/DPD ratio has a predictive value for disease-free survival in adjuvant 5-FU treated colorectal cancer patients.

The following examples, references, and figures are provided to aid the understanding of the present invention, the true scope of which is set forth in the appended claims.

### Description of the Figures

**Figure 1 TP, DPD and TS mRNA expression in microdissected tissues.** Normal colonic mucosa (n=8) and muscularis propia (n=3), chronic colitis (n=3), colorectal cancer (CRC; n=102) and normal liver (n=1, duplicate). mRNA levels are expressed as relative ratio (mean +/- stdev.;).
**Figure 2 TP, DPD and TS mRNA expression in 102 CRC patients.** Each symbol reflects one case, with bars and numbers indicating median mRNA levels (relative ratio) for: all cases (squares, n=102), "no CTX" (triangles, n=40) and "CTX" (circles, n=52).
**Figure 3 Association of TP, DPD and TS mRNA expression with tumor histology.** Graphical overview of the statistically significant correlations, with columns representing the median level within each sub-group. Y-axis denotates relative mRNA levels or enzyme ratios. For p-values refer to Table 2.
**Figure 4 Correlation of TP/DPD ratio with overall and disease-free survival.** Kaplan-Meier analysis with respect to overall survival for the "no CTX" (n=40) and "CTX" (n=52) groups (A). Neither TP, DPD nor TS mRNA levels were of predictive value for overall survival in 5-FU treated ("CTX") patients (B, with cut-off = median mRNA expression). However, low DPD mRNA levels ratio were significantly correlated to disease-free survival in 5-FU treated patients (C, with cut-offs as indicated).
**Figure 5 Correlation of TP/DPD ratio mRNA levels with overall and disease-free survival.** Kaplan-Meier analysis with respect to disease free survival and its correlation with the TP/DPD ratio. High TP/DPD ratios were significantly correlated to disease-free survival in 5-FU treated patients (Cut off values: A=0.39, B=3.7, C=5.0, D=6,2, E=8.1).

### Examples

### Example 1

### Patients and tissues.

Colorectal cancer specimens were obtained from the archive of the Institute of Pathology, Klinikum rechts der Isar, Munich, Germany. Resected primary tumor specimens from a total of 102 patients (Table 1; median clinical follow up = 63.5 months, range 8-125 months), were analysed after microdissection of tumor cells.

**Table 1: Summary of patient characteristics.**

| | | All | | No CTX¹ | | CTX¹ | |
|---|---|---|---|---|---|---|---|
| | | No. | % | No. | % | No. | % |
| Patients | | 102 | | 40 | | 52 | |
| Age: | | 62 yrs | | 68 yrs | | 60 yrs | |
| Sex: | Male | 67 | 65.7 | 25 | 62.5 | 35 | 67.3 |
| | Female | 35 | 34.3 | 15 | 37.5 | 17 | 32.7 |
| Tumor Site: | | | | | | | |
| Colon | | 65 | 63.7 | 21 | 52.5 | 42 | 80.8 |
| Rectum | | 37 | 36.3 | 19 | 47.5 | 10 | 19.2 |
| T Category: | T1 | 4 | 3.9 | 3 | 7.5 | 1 | 1.9 |
| | T2 | 14 | 13.7 | 9 | 22.5 | 5 | 9.6 |
| | T3 | 64 | 62.8 | 26 | 65 | 29 | 55.8 |
| | T4 | 20 | 19.6 | 2 | 5 | 17 | 32.7 |
| | N Category: | | | | | | |
| | N0 | 52 | 51 | 38 | 95 | 4 | 7.7 |
| | N1 | 32 | 31.4 | 1 | 2.5 | 31 | 59.6 |
| | N2 | 18 | 17.6 | 1 | 2.5 | 17 | 32.7 |
| UICC Stage: | I | 12 | 11.8 | 12 | 30 | - | - |
| | II | 40 | 39.2 | 26 | 65 | 4 | 7.7 |
| | III | 50 | 49 | 2 | 5 | 48 | 92.3 |
| Diff. Grade: | 2 | 70 | 68.6 | 31 | 77.5 | 31 | 59.6 |
| | 3 | 31 | 30.4 | 9 | 22.5 | 20 | 38.5 |
| | 4 | 1 | 1 | - | - | 1 | 1.9 |
| Clinical Data | | | | | | | |
| Recurrent disease | | 33 | 32.4 | 11 | 27.5 | 22 | 42.3 |
| Disease free survival² | | 59 mo. | | 64 mo. | | 56 mo. | |
| Overall survival² | | 63.5 mo. | | 65.5 mo. | | 57 mo. | |
| Follow up² | | 63.5 mo. | | 65.5 mo. | | 57 mo. | |
| | | (8-125) | | (14-125) | | (8-125) | |

| | | | | | | | |
|---|---|---|---|---|---|---|---|
| ¹ = 10 cases excluded from statistical analysis due to combination therapies; ² = numbers represent the statistical median (range) of survival in months. | | | | | | | |

40 patients underwent tumor resection only ("no CTX" group), 52 patients had received adjuvant chemotherapy after resection ("CTX" group) and 10 patients had received a combined adjuvant chemo- / radiotherapy. The adjuvant regimens in the "CTX" group were as follows: 25/52 patients Mayo protocol (6 months of 425mg/m² 5-FU and 20mg /m² leucovorin) [O'Connell, M.J., et al., J. Clin. Oncol. 15 (1997) 246-250], 7/52 "Mörtel" regimen [450 mg/m² 5-FU and 50 mg/m² levamisol) [Moertel, C.G., et al., N. Engl. J. Med. 322 (1990) 352-358], 5/52 patients Ardalan protocol (24 hours of 2,600mg/m² 5-FU and 500mg/m² Leucovorin) [Ardalan, B., et al., J. Clin. Oncol. 9 (1991) 625-630], 14/52 patients modified Ardalan protocol and 1/52 patients SAKK protocol (500mg/m² 5-FU and 10mg/m² mitomycin C) [SAKK, Lancet 345 (1995) 349-353]. For control purposes, tissue specimens of normal colon, chronic colitis (see below) and normal liver were obtained. All tissues had been formalin-fixed (10% buffered formalin, 24 hrs) and paraffin-embedded (FFPE) according to routine guidelines. Before analysis, histopathology of each specimen was confirmed on hematoxylin stained serial sections.

### Example 2

### Microdissection.

Prior to RNA extraction from FFPE-tissues, microtom sections (5µm) were treated with xylene and graded alcohols under RNase-free conditions. For subsequent microdissection, sections were individually stained with instant hematoxylin (Shandon, Frankfurt, Germany) and tumor cells were dissected under microscopic observation using fine needles (gauge 18). The purity of the dissected tumor cell population was 80-90%.

Control tissues were dissected under equal conditions and included normal colonic mucosa (n=8; epithelial cells) and colonic muscularis propria (n=4; muscle cells), reactive lesions of chronic colitis (n=3; Crohn's disease, ulcerative colitis and diverticulitis of the sigmoid) and normal liver (n=1; all cell populations). For the latter two control groups, duplicate analysis was performed by processing two serial sections of each tissue specimen separately.

### Example 3

### RNA Isolation.

In 52 CRC cases, microdissected tissue samples were subjected to RNA isolation as described previously[Lassmann, S., et al., J. Pathol. 198 (2002) 198-206]. In brief, microdissected tumour cells were immediatly placed into Eppendorf tubes, containing digestion buffer (10 mM TrisHCl, 0.1 mM EDTA, 2 % SDS and 0.5 mg Proteinase K, all from Sigma, Taufkirchen, Germany). Incubation was overnight (60°C, 350-400 rpm), followed by phenol:choroform extraction and precipitation of nucleic acids in isopropanol. The resulting RNA pellet was further purified by incubation (45 min, 37°C) with 10 U DNase I (Roche Diagnostics GmbH, Mannheim, Germany), 20 µl DNase buffer (0.4 M TrisHCl, 60 mM MgCl₂, 0.1 M NaCl) and H₂O up to 200 µl. Thereafter RNA was re-extracted by phenol:chloroform extraction, precipitation and resuspension in H₂O. In 50 CRC cases and the control specimens, microdissected tumor or control cells were isolated with the "HighPure RNA Paraffin Kit" (Roche Diagnostics GmbH, Mannheim, Germany) according to the supplied protocol. This method also consists of a Proteinase K digestion step, purification of nucleic acids, a DNase digestion step and re-purification of the RNA. In preliminary experiments similar results were obtained from 3 serial tissue sections of each a normal mucosa and a tumor sample isolated by the two methods (data not shown). RNA was stored at - 70°C until further use.

### Example 4

### Reverse-Transcription and Quantitative Polymerase Chain Reaction (QRT-PCR).

Reverse transcription and quantitative PCR in the LightCycler^{®} system was performed with reagents and kits from Roche Applied Science according to the supplier's instructions. In brief, RNA samples were distributed to four equal aliquots, all receiving the same mix of cDNA reagents and either TP, DPD, TS or reference gene specific primers (Cat. Nos. 3 302 946, 3 302 938, 3 302 954). A positive control RNA (calibrator, from the "LC - mRNA quantification Kits for TP, DPD and TS") was included in this step. Always one calibrator RNA together with 4 unknown samples was treated as a separate "set" in order to control for quality and reproducibility. Always 3 cDNA "sets" were then analysed together in one run of quantitative PCR, using the "LC - mRNA quantification kits for TP, DPD and TS". For data analysis, the "Relative Quantification Software" (Roche Diagnostics GmbH, Mannheim, Germany) was applied. This calculates the relative ratio of Cp(enzyme : reference gene)ₛₐₘₚₗₑ to Cp(enzyme : reference gene)_{calibrator}, thereby controling both for sample loading (RNA input) and PCR efficiency due to the constant reference point (calibrator). To ensure accurate quantification, only data of RNA preparations with crossing points of 20 to max. 33 (linear amplification range) were included. The variance of TP, DPD, TS and reference gene mRNA expression (mean +/- stdev. of crossing point) was minimal, with 26.62 +/- 0.36, 28 +/-0.51, 22 +/-0.23 and 23.19 +/-0.7 for 29 calibrators (accounting for 29 x 4 = 116 tissue samples), respectively.

### Example 5

### Statistics.

Quantitative parameters were described using mean or median with standard deviation and ranges, respectively. Qualitative parameters were examined by frequency tables. Non-parametric tests were performed (SAS^{®} software; version 8.02), as a deviation from the normal distribution was observed for all markers.

In the group of all 102 cases, TP, DPD and TS mRNA expression as well as the TS/DPD and TP/DPD ratio were correlated to 1) patient age and gender, and 2) primary tumor localisation, TNM classification, UICC stage and differentiation grade. This was done by the Spearman correlation coefficient and the test on zero correlation. For comparison of individual subgroups, the Wilcoxon-test for unpaired samples and the Kruskal-Wallis-test were applied. In order to evaluate the prognostic impact and / or response prediction value of the three enzymes, the 102 cases were divided into the "no CTX" (n=40) and the "CTX" (n=52) group. Patients who had received a combined radio- / chemotherapy (n=10) were excluded. Within the two subgroups separate statistical analysis was performed for correlation of TP, DPD and TS mRNA expression and the TS/DPD and TP/DPD ratios with: incidence of recurrent disease, incidence of death as well as disease-free and overall survival. The survival analysis was achieved by both a Cox-regresssion and a log-rank-test, setting the significance level to 5%.

### Example 6

### Results

### TP, DPD and TS mRNA expression in normal colon, chronic cholitis and CRC

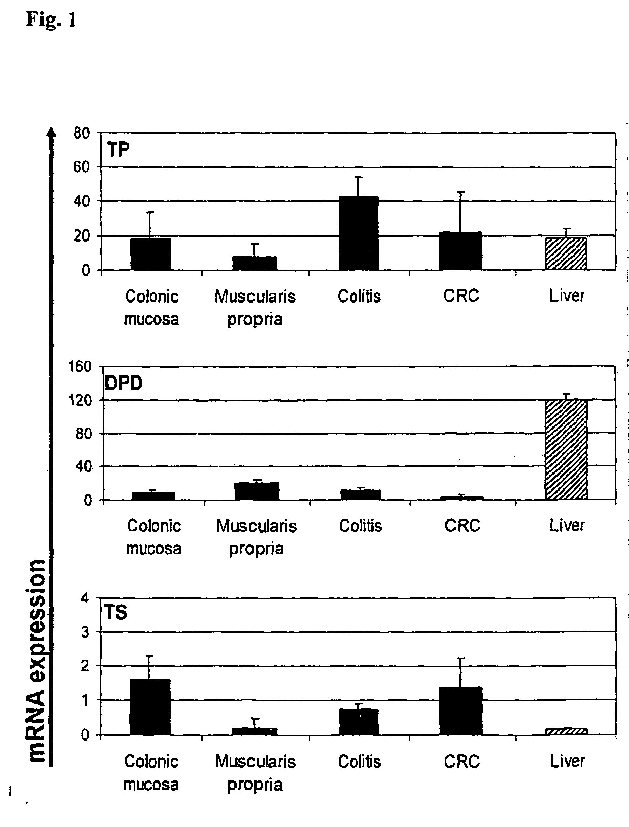

Initially, TP, DPD and TS mRNA levels were examined in a series of microdissected, FFPE control specimens by quantitative RT-PCR using the LightCycler^{®} system. As shown in Figure 1, mRNA expression for TP, DPD and TS was detected in all of the samples, but mRNA expression levels differed between tissues: High TP mRNA expression was seen in reactive lesions of chronic colitis, followed by moderate levels in normal colonic mucosa (epithelial cells) and normal liver (mixed cell population) and even lower expression in normal colonic muscularis propria (muscle cells). DPD mRNA expression was greatest in normal liver, followed by normal muscularis propria > reactive lesions of chronic colitis >/= normal mucosa. TS was highly expressed in normal mucosa > reactive lesions of chronic colitis > normal liver and muscularis propria.

In comparison, the mean TP, DPD and TS mRNA expression levels of colon tumor samples (n=102, see below) revealed a lower DPD mRNA expression in tumor samples when compared to epithelial cells of normal mucosa. In contrast, no significant difference of TP and TS mRNA levels was detected between normal mucosa and tumor tissues.

### Screening of TP, DPD and TS mRNA expression in 102 patients with colorectal cancer

The group of patients examined included 102 cases of CRC of various stages (Table 1, *Materials and Methods*), either treated with resection alone (40 cases; "no CTX" group), with resection and subsequent 5-FU chemotherapy (52 cases; "CTX" group) or with resection and a combination of radio- and chemotherapy (10 cases).

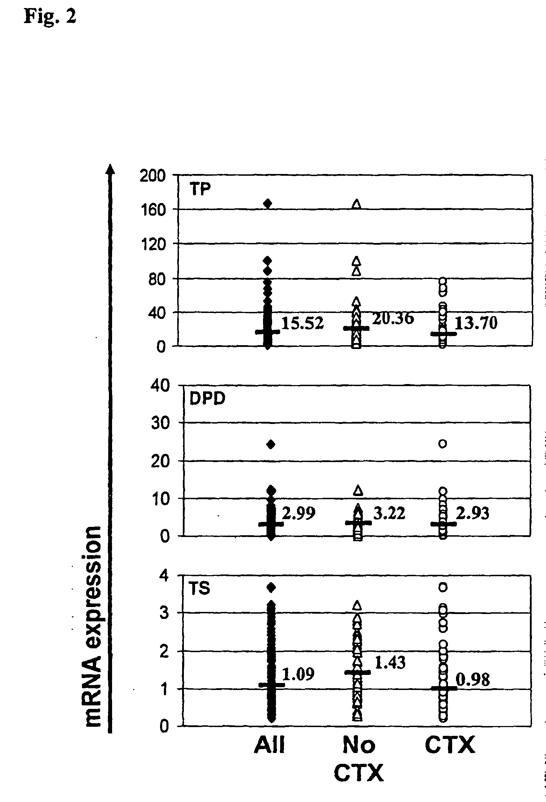

Analysis of the mRNA expression ofTP, DPD and TS in all 102 CRC cases showed a wide range of enzyme expression patterns (Figure 2). Expressed as a relative ratio, the ranges for TP, DPD and TS were 1.52-166.29, 0-24.39 and 0.21-3.71, respectively. Upon division of the cases into groups of "no CTX" and "CTX", TP, DPD and TS mRNA expression was similar in both groups, except for a trend to lower TP mRNA expression in the "CTX" group. This is reflected by the statistical median of TP, DPD and TS mRNA expression levels (Figure 2).

### Correlation of TP, DPD and TS mRNA expression to patient data and histology

As summarized in Table 2A, no statistically significant correlation was revealed between TP, DPD or TS mRNA levels or the TS/DPD and TP/DPD ratios with patient age or gender and the location of the primary tumor (colon or rectum).

**Table 2: Numbers represent p-values of Kruskal-Wallis test for patient and tumor parameters**

| *A* | | TP | DP D | TS | TP/DPD ratio | TS : DPD ratio |
|---|---|---|---|---|---|---|
| Patient | | | | | | |
| Age | | - | - | - | - | - |
| Gender | | - | - | - | | - |
| Tumor | Location | - | - | - | - | - |
| | T Category | 0.03 | - | - | 0.007 | 0.014 |
| | N Category | 0.04 | - | - | 0.001 | - |
| | UICC Stage | 0.009 | - | - | 0.001 | - |
| | Diff. Grade | - | - | 0.0014 | - | 0.033 |

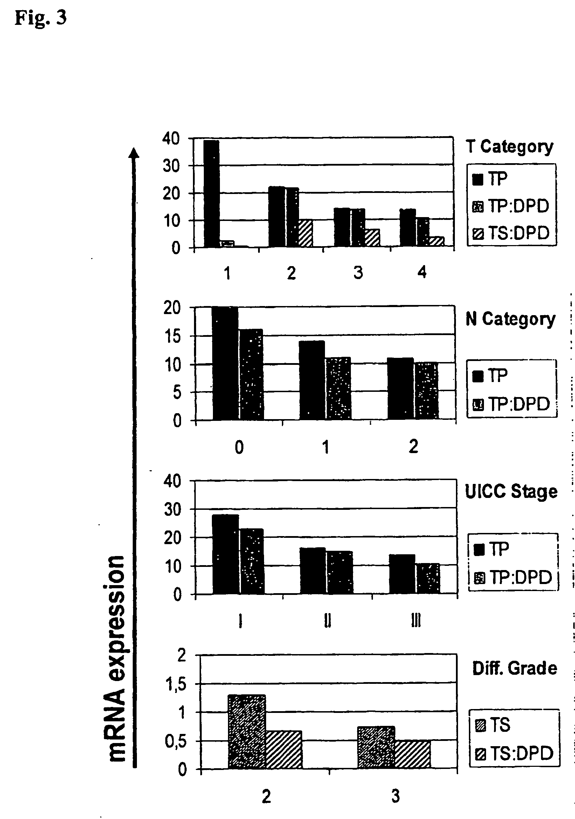

However, significant differences were seen with respect to 1) TP mRNA expression with tumor T (p=0,03) and N (p=0,04) category and UICC stage (p=0,009); 2) TS mRNA expression with differentiation grade (p=0,001), 3) the TS/DPD ratio with tumor T category (p=0.014) and differentiation grade (p=0.033) and 4) the TP/DPD ratio with tumor T (p=0,007) and N (p=0,001) category and UICC stage (p=0,001). As shown in Figure 3, TP mRNA expression and the TP/DPD ratio significantly decreased with higher tumor T and N categories as well as with higher UICC stages. TS mRNA expression was significantly lower in differentiation grade 3 than grade 2 tumors. Finally, the TS/DPD ratio was lower in tumors with higher T category as well as in differentiation grade 3 than grade 2 tumors.

### TP, DPD and TS mRNA expression in CRC - correlation to prognosis

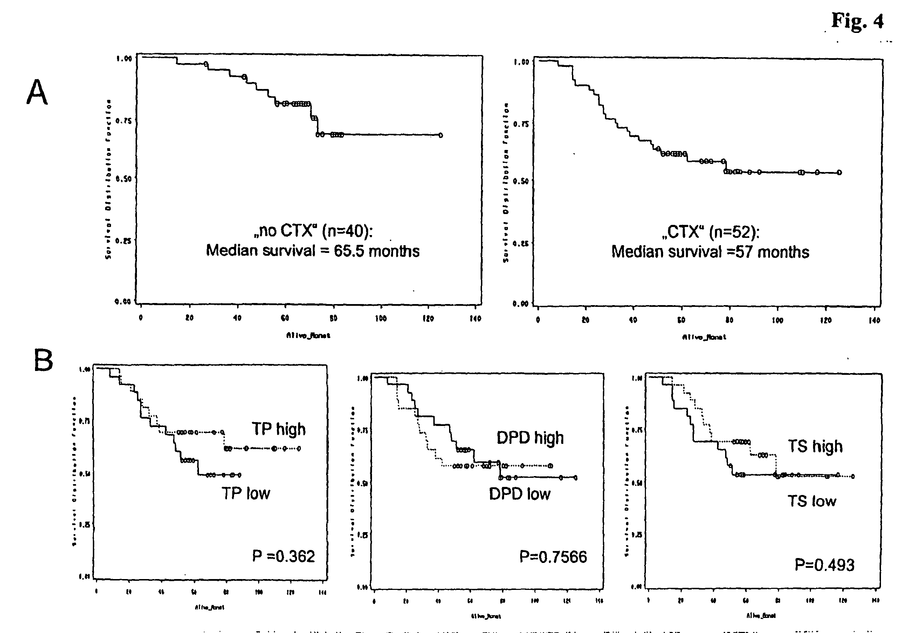

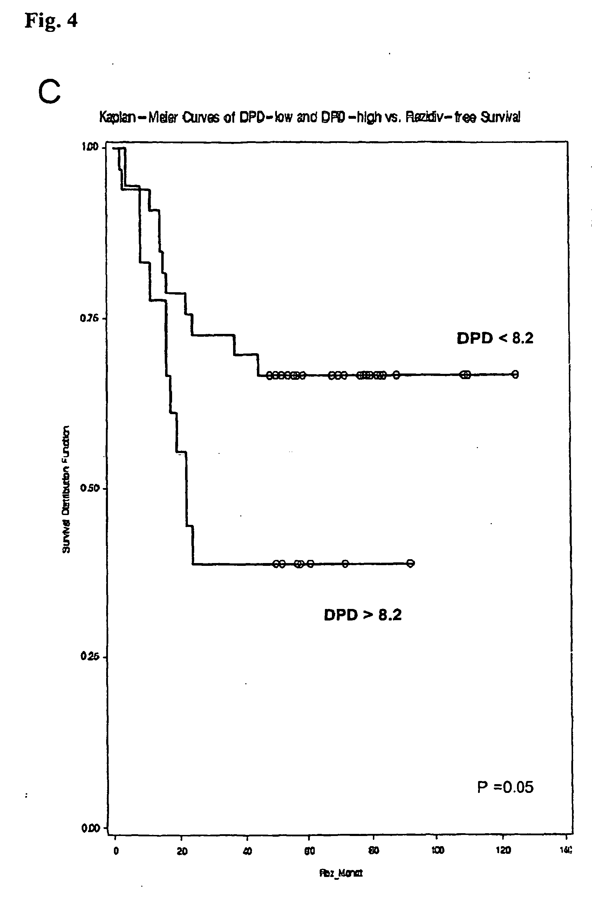

In order to correlate TP, DPD and TS mRNA expression with prognosis, patients who had received adjuvant chemo- and radiotherapy (n=10) were excluded from statistical analysis. Moreover, as patients with lymphnode involvement ("N+") have in general a poorer prognosis than those who are classified as "N0" [1], the remaining 92 patients were divided into those without adjuvant therapy (n=40; "no CTX") and those with adjuvant chemotherapy (n=52; "CTX"), as shown in Figure 4A. Statistical analysis was then performed separately within the two groups with respect to incidence of recurrent disease and death as well as disease-free and overall survival. First, no significant correlation was revealed between either TP, DPD or TS mRNA expression or the TS/DPD and TP/DPD ratios and the incidence of recurrent disease or death (Table 2B).

**Table 2: Numbers represent p-values of Cox regression for follow-up parameters**

| B | **TP** | **DPD** | **TS** | **TP/DPD** ratio | **TS : DPD** ratio |
|---|---|---|---|---|---|
| "No CTX" Group | | | | | |
| Recurrent Disease | - | - | - | - | - |
| Overall survival | - | - | - | - | 0.032 |
| "CTX" Group | | | | | |
| Recurrent Disease | - | - | - | - | - |
| Disease-free survival | - | 0.05 | - | 0.002 | - |
| Overall survival | - | - | - | - | - |

Second, none of the enzymes or the TP/DPD ratio had a significant influence on overall survival (Kaplan-Meier analysis). Third, multivariate analysis of TP, DPD and TS mRNA expression and overall survival did not reveal any significant correlation, even though TP and DPD mRNA expression were significantly (p <0.0001) correlated in the "CTX" group. These findings were true for both the "no CTX" or "CTX" group (Table 2).

In contrast, within the "no CTX" group overall survival significantly correlated to the TS/DPD ratio (p=0.032), whereby the risk of death increases with higher TS/DPD ratios.

### TP, DPD and TS mRNA expression - markers for response prediction

To evaluate whether TP, DPD and/or TS mRNA levels or the ratio of TP/DPD or TS/DPD can predict the clinical response to 5-FU chemotherapy, detailed statistical analysis was performed for patients within the "CTX" group. Upon sub-division of the "CTX" patients into those with "low" and "high" TP, DPD and TS mRNA levels (cut off =median, as indicated in Figure 2) and subsequent Kaplan Meier analysis, no correlation was found with respect to overall survival (Figure 4B). Similarly, neither low or high TP/DPD or TS/DPD ratios did predict for overall survival in the two "CTX" sub-groups (not shown).

However, significant correlations were seen for DPD mRNA levels and the TP/DPD ratio with respect to disease-free survival (Figure 4C). Thus, using a cut-off mRNA level of 8.2, low DPD mRNA expression was correlated to disease-free survival with p = 0.05.

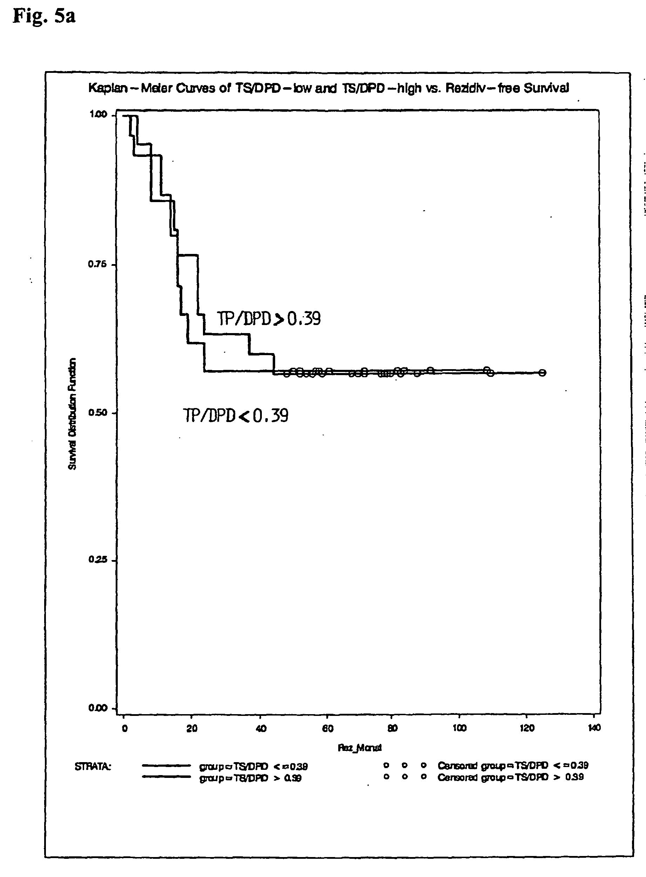

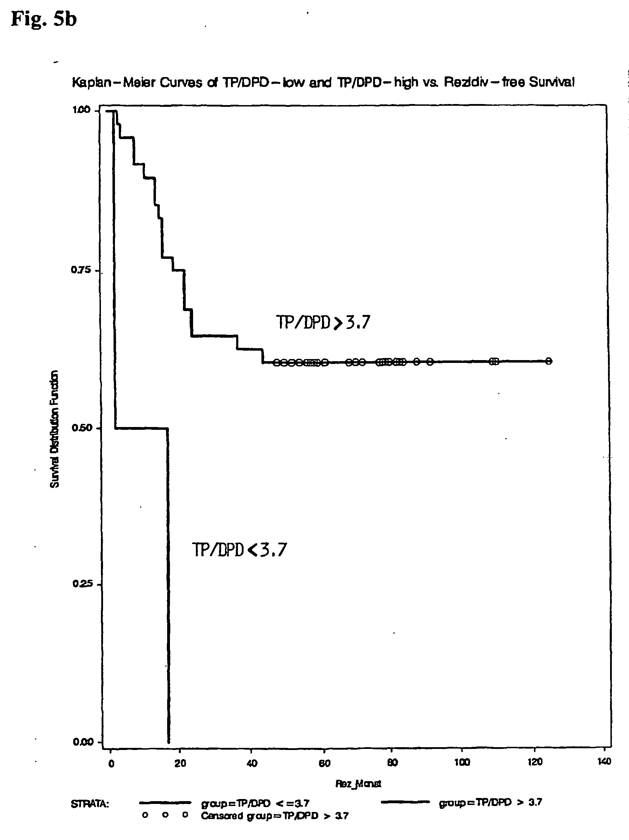

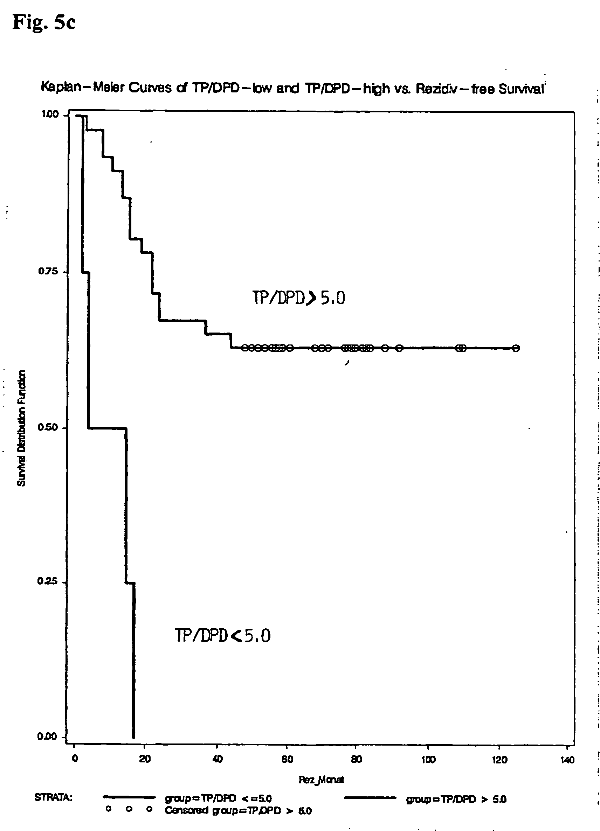

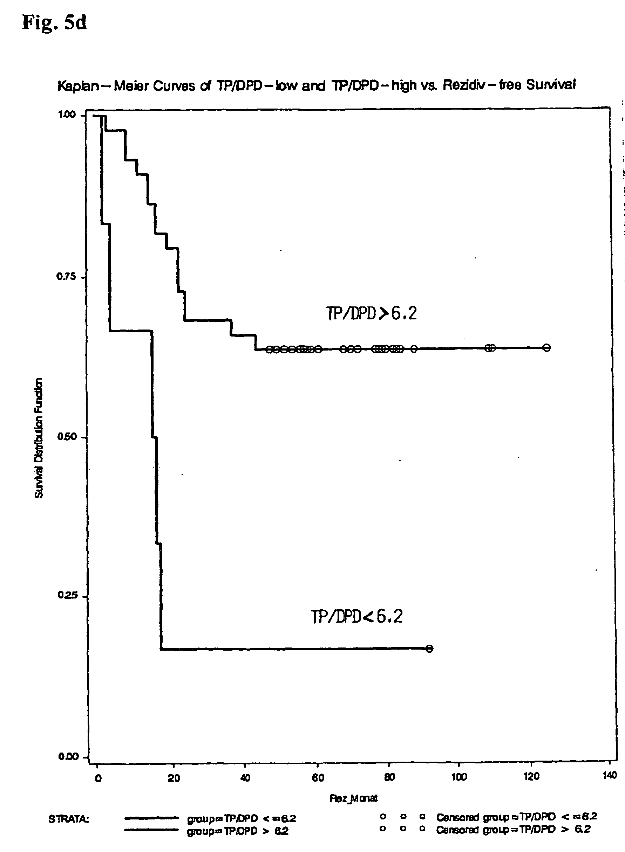

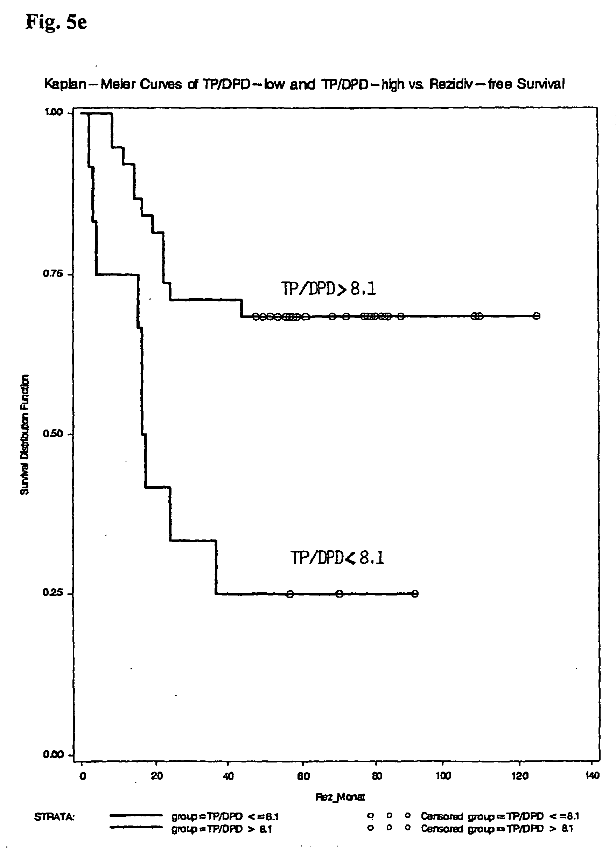

Moreover, a positive correlation between disease free survival and TP/DPD ratio has been identified. As it is shown in Figure 5, using cut-off values of 3.7 (fig. 5b), 5.0 (fig. 5c), 6.2 (fig. 5d), and 8.1 (fig. 5e), a high TP/DPD ratio was significantly correlated to disease-free survival with p =0.002.

### List of References

Allegra, C.J., et al., J. Clin. Oncol. 20 (2002) 1735-1743
Araki, Y., et al., Kurume Med. J. 48 (2001) 93-98
Ardalan, B., et al., J. Clin. Oncol. 9 (1991) 625-630
Backus, H.H., et al., J. Clin. Pathol. 55 (2002) 206-211
Bathe, O.F., et al., Cancer J. Sci. Am. 5 (1999) 34-40
Copur, S., et al., Biochem. Pharmacol. 49 (1995) 1419-1426
Diasio, R.B., and Johnson, M.R., Pharmacology 61 (2000) 199-203
Edler, D., et al., J. Clin. Oncol. 20 (2002) 1721-1728
Findlay, M.P., et al., Br. J. Cancer 75 (1997) 903-909
Fox, S.B., et al., J. Pathol. 176 (1995) 183-190
Fujiwaka R. et al, of Clinical Oncology (2000) 3946-3951
Furukawa, T., et al., Nature 356 (1992) 668
Griffiths, L, and Stratford, I.J., Br. J. Cancer 76 (1997) 689-693
Guimbaud, R., et al., Cancer Chemother. Pharmacol. 45 (2000) 477-482
Iacopetta, B., et al., Br. J. Cancer 85 (2001) 827-830
Iqbal, S., and Lenz, H.J., Curr. Oncol. Rep. 3 (2001) 102-108
Ishikawa, T., et al., Cancer Res. 58 (1998) 685-690
Johnson, M.R., et al., Clin. Cancer Res. 5 (1999) 2006-2011
Johnston, P.G., et al., Cancer Res. 55 (1995) 1407-1412
Johnston, S.J., et al., Clin. Cancer Res. 5 (1999) 2566-2570
Kawakami, K., et al., Clin. Cancer Res. 7 (2001) 4096-4101
Kornmann, M., et al., J. Gastrointest. Surg. 6 (2002) 331-337
Kumar, S.K., and Goldberg, R.M., Curr. Oncol. Rep. 3 (2001) 94-101
Lassmann, S., et al., J. Pathol. 198 (2002) 198-206
Marsh, S., et al., Int. J. Oncol. 19 (2001) 383-386
Metzger, R., et al., Clin. Cancer Res. 4 (1998) 2371-2376
Miwa, M., et al., Eur. J. Cancer 34 (1998) 1274-1281
Miyamoto, S., et al., Int. J. Oncol. 18 (2001) 705-713
Moertel, C.G., et al., N. Engl. J. Med. 322 (1990) 352-358
Mori, K., et al., Int. J. Oncol. 17 (2000) 33-38
O'Connell, M.J., et al., J. Clin. Oncol. 15 (1997) 246-250
Paradiso, A., et al., Br. J. Cancer 82 (2000) 560-567
Pestalozzi, B.C., et al., Br. J. Cancer 71 (1995) 1151-1157
Pestalozzi, B.C., et al., Br. J. Cancer 71 (1995) 1151-1157
Ragnhammar, P., et al., Acta Oncol. 40 (2001) 282-308
Raida, M., et al., Clin. Cancer Res. 7 (2001) 2832-2839
SAKK, Lancet 345 (1995) 349-353
Salonga, D., et al., Clin. Cancer Res. 6 (2000) 1322-1327
Sanguedolce, R., et al., Anticancer Res. 18 (1998) 1515-1520
Sumizawa, T., et al., J. Biochem. 114 (1993) 9-14
Takebayashi, Y., et al., Eur. J. Cancer 32 (1996) 1227-1232
Takebayashi, Y., et al., J. Natl. Cancer Inst. 88 (1996) 1110-1117 Takenoue, T., et al., Ann. Surg. Oncol. 7 (2000) 193-198
Tanaka-Nozaki, M., et al., Clin. Cancer Res. 7 (2001) 2783-2787
Terashima M. et. al., Europ. J. of Cancer (2002) 2375-2381
Uetake, H., et al., Clin. Cancer Res. 5 (1999) 2836-2839
van Kuilenburg, A.B., et al., Clin. Cancer Res. 6 (2000) 4705-4712
Wang, W., et al., Cancer Res. 61 (2001) 5505-5510
Wei, X., et al., J. Clin. Invest. 98 (1996) 610-615
WO 02/44423

## Claims

1. Method for determining whether a patient suffering from colorectal cancer is susceptible to a treatment with a 5-Fluoro-Uracil and/or 5-FU analogon comprising
a) determination of Thymidine Phosphorylase mRNA expression in a clinical sample
b) determination of Dihydropyrimidine Dehydrogenase mRNA expression in said clinical sample
c) determination of the ratio of the value obtained in step a) and the value obtained in step b)
d) determination whether the ratio obtained in step c exceeds a predetermined cut off value

2. Method according to claim 1, wherein said cut off value is at least 3 and preferably at least 3.7

3. Method according to claim 2, wherein said cut off value is not higher than 10 and preferably not higher than 8.2

4. Method according to claims 1-3, further **characterized in that** an absolute or relative mRNA expression level of Dihydropyrimidine Dehydrogenase is determined and an expression value below a certain cut off value is additionally indicative for susceptibility to a 5 Fluoro-Uracil and/or 5-FU analogon.

5. Method according to claims 1-4, **characterized in that** mRNA expression of Thymidine Phosphorylase and/or Dihydropyrimidine Dehydrogenase is determined by means of Reverse Transciptase PCR.

6. Method according to claim 5, **characterized in that** a sequence specific Thymidine Phosphorylase and/or a sequence specific Dihydropyrimidine Dehydrogenase primer is elongated during the cDNA synthesis step.

## Patentansprüche

1. Verfahren zur Bestimmung, ob ein Patient, der an Darmkrebs leidet, auf eine Behandlung mit 5-Fluor-Uracil und/oder einem 5-FU Analogon anspricht, umfassend:
a) Bestimmung der Thymidin-Phosphorylase mRNA-Expression in einer klinischen Probe
b) Bestimmung der Dihydropyrimidin-Dehydrogenase mRNA-Expression in der klinischen Probe
c) Bestimmung des Verhältnisses des in Schritt a) erhaltenen Wertes und des in Schritt b) erhaltenen Wertes
d) Bestimmung, ob das in Schritt c) erhaltene Verhältnis einen vorbestimmten Grenzwert übersteigt.

2. Verfahren nach Anspruch 1, worin der Grenzwert mindestens 3 und bevorzugt mindestens 3,7 beträgt.

3. Verfahren nach Anspruch 2, worin der Grenzwert nicht höher als 10 und bevorzugt nicht höher als 8,2 ist.

4. Verfahren nach einem der Ansprüche 1-3, weiter **dadurch gekennzeichnet, dass** ein absoluter oder relativer mRNA-Expressionswert der Dihydropyrimidin-Dehydrogenase bestimmt wird, und dass ein Expressionswert unterhalb eines bestimmten Grenzwerts ein zusätzliches Anzeichen für eine Empfindlichkeit gegenüber 5-Fluor-Uracil und/oder einem 5-FU Analogon ist.

5. Verfahren nach einem der Ansprüche 1-4, **dadurch gekennzeichnet, dass** die mRNA-Expression von Thymidin-Phosphorylase und/oder Dihydropyrimidin-Dehydrogenase mittels Reverse-Transkriptase PCR bestimmt wird.

6. Verfahren nach Anspruch 5, **dadurch gekennzeichnet, dass** ein sequenzspezifischer Thymidin-Phosphorylase Primer und/oder ein sequenzspezifischer Dihydropyrimidin-Dehydrogenase Primer während des cDNA Syntheseschritts verlängert wird.

## Revendications

1. Procédé pour déterminer si un patient souffrant d'un cancer colorectal est sensible au traitement avec un 5-fluorouracile et/ou un analogue de 5-FU, comprenant
a) la détermination de l'expression d'ARNm de thymidine-phosphorylase dans un échantillon clinique
b) la détermination de l'expression d'ARNm de dihydropyrimidine-déshydrogénase dans ledit échantillon clinique
c) la détermination du rapport de la valeur obtenue dans l'étape a) et de la valeur obtenue dans l'étape b)
d) la détermination du fait que le rapport obtenu dans l'étape c) est ou non supérieur à une valeur de seuil prédéterminée.

2. Procédé selon la revendication 1, dans lequel ladite valeur de seuil est au moins 3 et de préférence au moins 3,7.

3. Procédé selon la revendication 2, dans lequel ladite valeur de seuil n'est pas supérieure à 10 et de préférence pas supérieure à 8,2.

4. Procédé selon les revendications 1-3, **caractérisé en outre en ce que** l'on détermine un niveau d'expression absolu ou relatif d'ARNm de dihydropyrimidine-déshydrogénase et qu'une valeur d'expression inférieure à une certaine valeur de seuil indique en plus une sensibilité à un 5-fluorouracile et/ou un analogue de 5-FU.

5. Procédé selon les revendications 1-4, **caractérisé en ce que** l'on détermine l'expression d'ARNm de thymidine-phosphorylase et/ou de dihydropyrimidine-déshydrogénase au moyen d'une PCR avec transcriptase inverse.

6. Procédé selon la revendication 5, **caractérisé en ce qu'**une amorce de thymidine-phosphorylase spécifique de la séquence et/ou une amorce de dihydropyrimidine-déshydrogénase spécifique de la séquence est allongée au cours de l'étape de synthèse d'ADNc.
